# EUROPÄISCHE PATENTSCHRIFT

(11) **EP 1 825 843 B1**
(45) Veröffentlichungstag und Bekanntmachung des Hinweises auf die Patenterteilung: **10.06.2009**
(21) Anmeldenummer: 06110429.5
(22) Anmeldetag: 24.02.2006
(51) Int. Cl.: A61K 6/083

(54) **Dentalmaterialien auf der Basis von multicyclischen Allylsulfiden**
Dental material based on multicyclic allyl sulfides
Matériau dentaire à base de sulfures allyliques multicycliques

(43) Veröffentlichungstag der Anmeldung: 29.08.2007
(73) Patentinhaber: Ivoclar Vivadent AG, FL-9494 Schaan (LI)
(72) Erfinder: Angermann, Dr., Jörg, CH-7320 Sargans (CH); Burtscher, Dr., Peter, A-6830, Rankweil (AT); Fischer, Urs Karl, CH-9320, Arbon (CH); Moszner, Prof. Dr., Norbert, FL-9495, Triesen (LI); Rheinberger, Dr., Volker, FL-9490, Vaduz (LI)
(74) Vertreter: UEXKÜLL & STOLBERG

(56) Entgegenhaltungen:
- WO-A-96/19471

## Beschreibung

Die Erfindung betrifft Dentalwerkstoffe auf der Basis von radikalisch polymerisierbaren, multicyclischen Allylsulfiden. Die erfindungsgemäßen Dentalmaterialien eignen sich besonders als Komposite, Zemente, Adhäsive, zur Herstellung von Beschichtungen oder dentalen Formkörpern.

Radikalisch polymerisierbare cyclische Monomere sind aufgrund des im Vergleich zu linearen Monomeren deutlichen geringeren Polymerisationsschrumpfes von besonderem Interesse (vgl. R. K. Sadhir, R. M. Luck, Expanding Monomers, CRC Press, Boca Raton etc. 1992).

Die WO 96/19471 offenbart mono- oder difunktionelle cyclische Allylsulfide, die sich zur Herstellung von Adhäsiven, dentalen Kompositen und optischen Linsen eignen sollen. Diese Monomere sollen bei der Polymerisation eine geringere Schrumpfung als herkömmliche Monomere zeigen. Leider zeichnen sich diese Monomeren durch eine im Vergleich zu kommerziellen Dimethacrylaten ungenügende, zu geringe Reaktivität bei der radikalischen Photopolymerisation aus.

R. A. Evans, E. Rizzardo, Macromolecules 29 (1996) 6983-6989, beschreiben die ringöffnende Polymerisation von 6-Methylen-1,3-dithiepan und 3-Methylen-1,5-dithiacyclooctan. Diese Monomere sollen gegenüber Feuchtigkeit sowie Säuren und Basen stabil sein, weisen jedoch einen durchdringenden, unangenehmen Geruch auf.

In späteren Arbeiten, R. A. Evans, E. Rizzardo, Macromolecules 33 (2000) 6722-6731 und R. A. Evans, E. Rizzardo, J. Polym. Sci.: Part A Polym. Chem. 39 (2001) 202-215, untersuchen dieselben Autoren den Einfluß von Substituenten auf die Polymerisation von mono- und bicyclischen Allylsulfiden. Bei unsymmetrisch substituierten Monomeren wurden amorphe Polymerisationsprodukte erhalten, die sich gegenüber kristallinen Produkten durch einen geringeren Schrumpf auszeichnen sollen. Das untersuchte bicyclische Monomer Bis(6-methylen-1,4-dithiacycloheptan-2-ylmethyl)diglycolat ergab ein gummiartiges, weiches Polymer, das für dentale Anwendungen ungeeignet ist.

W. Weinmann, C. Thalacker, R. Guggenberger, Dent. Mat. 21 (2005) 68-74, beschreiben Dentalmaterialien auf der Basis von mit cycloaliphatischen Epoxiden modifizierten Cyclosiloxanen, sogenannten Siloranen, die eine weitere Verringerung des Polymerisationsschrumpfes ermöglichen sollen. Nachteilig an diesen Verbindungen ist jedoch ihre Empfindlichkeit gegenüber Säuren.

Der Erfindung liegt die Aufgabe zugrunde, Dentalmaterialien bereitzustellen, die bei der radikalischen Polymerisation eine mit auf Dimethacrylaten basierenden Materialien vergleichbare Reaktivität und nur einen geringen Schrumpf zeigen. Weiterhin sollen die Dentalmaterialien säurestabil sein, um saure Monomere einarbeiten zu können und so selbsthaftende Werkstoffe zugänglich zu machen.

Diese Aufgabe wird durch Dentalwerkstoffe gelöst, die mindestens ein multicyclisches Allylsulfid mit der allgemeinen Formel (I) enthalten 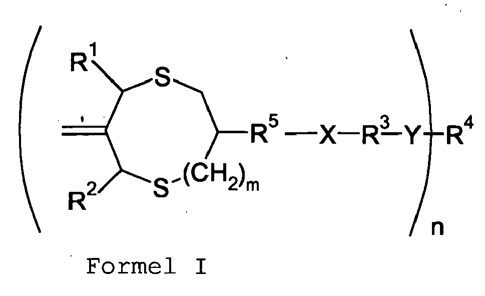 in der R¹ bis R⁴, X, Y, m und n unabhängig voneinander die folgenden Bedeutungen haben:
- R¹ =: H oder ein C₁-C₁₀-Alkyl-Rest,
- R² =: H oder ein C₁-C₁₀-Alkyl-Rest,
- R³ =: entfällt oder ein C₁-C₂₀-Alkylen-Rest, der durch O oder S unterbrochen sein kann, ein cycloaliphatischer C₄-C₁₂-Rest, ein bicyclischer C₆-C₁₂-Rest, ein C₆-C₁₄-Arylen- oder C₇-C₂₀-Alkylenarylen-Rest,
- R⁴ =: ein n-fach substituierter aliphatischer C₂- bis C₂₀-Kohlenwasserstoffrest, der durch O oder S unterbrochen sein kann, ein cycloaliphatischer C₄-C₁₂-Rest, ein aromatischer C₆-C₁₄-Rest, ein aliphatisch-aromatischer C₇-C₂₀-Rest oder ein heterocyclischer Rest, der 4 bis 20 Kohlenstoffatome und 1 bis 6 Heteroatome enthalten kann, die aus N-, O-, P- und/oder S-Atomen ausgewählt sind, oder der ausschließlich durch diese Heteroatome gebildet wird,
- R⁵ =: entfällt oder ein C₁-C₁₀-Alkylen-Rest,
- X =: entfällt, O, S, -O-CO- oder -O-CO-NH-,
- Y =: entfällt, O, S, -O-CO- oder -O-CO-NH-,
- m =: 0 oder 1 und
- n =: eine ganze Zahl von 3 bis 6.

Formel (I) erstreckt sich nur auf solche Verbindungen, die mit der chemischen Valenzlehre vereinbar sind.

Alkyl- und Alkylen-Reste können verzweigt oder vorzugsweise geradkettig sein.

Der Rest R⁴ ist n-fach durch die in Klammern stehende Molekülgruppe substituiert.

Der Hinweis, daß Gruppen durch Schwefel- oder Sauerstoffatome unterbrochen sein können, ist so zu verstehen, daß die Fremdatome in die Kohlenstoffkette der Gruppen eingeschoben werden, d.h. beidseitig von Kohlenstoffatomen gebunden werden. Die Fremdatome können somit keine endständige Position einnehmen. Werden mehrere Atome in eine Kohlenstoffkette integriert, müssen sie jeweils durch mindestens ein Kohlenstoffatom voneinander getrennt sein. Unter "Kohlenstoffkette" werden gerade und verzweigte Ketten nicht aber Ringe verstanden. Die Gesamtzahl der Atome, die in die Kohlenstoffkette integriert wird, ist mindestens um den Wert 1 kleiner als die Zahl der Kohlenstoffatome in der Kette.

Die Allylsulfide der Formel (I) lassen sich unter Ringöffnung radikalisch polymerisieren. Da die erfindungsgemäß verwendeten Allylsulfide mindestens drei polymerisierbare Gruppen, d.h. Allylsulfidringe aufweisen, haben sie vernetzende Eigenschaften. Die multicyclischen Allylsulfide werden im folgenden auch als multifunktionelle oder n-funktionelle Allylsulfide bezeichnet. Im Gegensatz zu anderen ringöffnenden Monomeren, wie methylengruppenhaltigen Spiroorthocarbonaten (SOC), Spiroorthoestern (SOE), bicyclischen Orthoestern (BOE) oder 1,1-disubstituierten 2-Vinylcyclopropanen (VCP) zeichnen sich die cyclischen Allylsulfide vor allem durch eine geringe Feuchtigkeitsempfindlichkeit und eine gute radikalische Copolymerisationsfähigkeit mit anderen Vinylmonomeren aus. Außerdem haben sie einen relativ hohen Brechungsindex, was für eine dentale Verwendung vorteilhaft ist.

Bevorzugt sind Dentalwerkstoffe die ein Allylsulfid gemäß Formel (I) enthalten, bei dem mindestens eine und vorzugsweise alle Variablen eine der folgenden Bedeutungen haben:
- R¹ =: H oder Methyl,
- R² =: H oder Methyl,
- R³ =: entfällt, ein C₁-C₆-Alkylen-Rest, ein cycloaliphatischer C₄-C₆-Rest oder ein C₆-C₁₂-Arylen-Rest,
- R⁴ =: ein n-fach substituierter aliphatischer C₂- bis C₁₀-Rest, ein cyclo-aliphatischer C₄-C₅-Rest, ein aromatischer C₆-C₁₂-Rest, ein heterocyclischer C₄-C₁₂-Rest, der 1 bis 6 Heteroatome enthalten kann, der Rest einer gesättigten oder ungesättigten Phosphor-Stickstoffverbindung, wie zB. ein cyclischer Phosphazan- oder Phosphazenrest,
- R⁵ =: entfällt oder ein C₁-C₃-Alkylen-Rest,
- X =: entfällt, O, -O-CO- oder -O-CO-NH-,
- Y =: entfällt, O, -O-CO- oder -O-CO-NH-,
- m =: 0 oder 1 und
- n =: 3 oder 4.

Ganz besonders bevorzugt sind Dentalwerkstoffe, die ein Allylsulfid gemäß Formel (I) enthalten, bei dem mindestens eine und vorzugsweise Variablen eine der folgenden Bedeutungen haben:
- R¹ =: H oder Methyl,
- R² =: H oder Methyl,
- R³ =: entfällt oder ein C₁-C₆-Alkylen-Rest,
- R⁴ =: ein n-fach substituierter aliphatischer C₂- bis C₆-Rest, ein aromatischer C₆-C₁₀-Rest, ein Cyanursäure- oder vorzugsweise Isocyanursäure-Rest (symmetrisches oder asymmetrisches Hexamethylendiisocyanattrimer), ein cyclischer Triphosphazenrest,
- R⁵ =: entfällt,
- X =: O oder -O-CO-NH-,
- Y =: entfällt oder O,
- m =: 0 oder 1 und
- n =: 3 oder 4.

Ein bevorzugter Phosphazanrest ist der Cyclotri(phosphazan)-Rest, ein bevorzugter Phosphazenrest der Cyclotri(phosphazen)-Rest.

Die n-funktionellen cyclischen Allylsulfide der allgemeinen Formel (I) können ausgehend von geeignet funktionalisierten monocyclischen Allylsulfiden durch bekannte Bindungsknüpfungsreaktionen mit entsprechend geeigneten n-funktionellen Precursorverbindungen erhalten werden. Die monocyclischen Allylsulfide, wie z.B. 6-Methylen-1,4-dithiepane (m = 0) oder 3-Methylen-1,5-dithiacyclooctane (m = 1) lassen sich durch Veretherung von geeigneten Dihalogenverbindungen mit Dithiolen, d.h. z.B. von 3-Chlor-2-chlormethyl-1-propen mit einem Ethan- oder Propandithiolderivat (HS-CH(R⁵-)-CH₂-SH bzw. HS-CH₂-CH(R⁵-)-CH₂-SH) analog zur Literatur (R. A. Evans, E. Rizzardo, J. Polym. Sci.: Part A Polym. Chem. 39 (2001) 202-215) herstellen:

### Konkretes Beispiel:

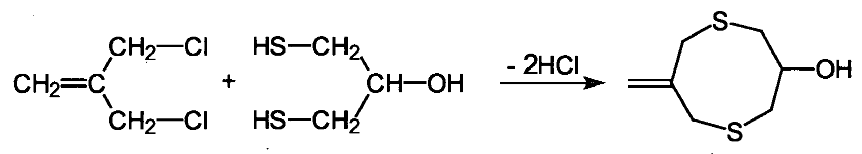

Andererseits lassen sich z.B. auch mit der Gruppe X' funktionalisierte 3-Methylen-1,5-dithiacyclooctane (m = 1) durch Veretherung von z.B. in 2-Position mit X'-funktionalisierten 1,3-Dichlorpropan Cl-CH₂-CH(R⁵-X')-CH₂-Cl mit 3-Mercapto-2-mercaptomethyl-1-propen (HS-CH₂-C(=CH₂)-CH₂-SH) synthetisieren (analog zu DD 100 001).

### Konkretes Beispiel:

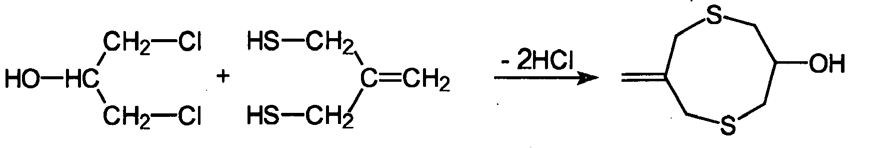

Bei funktionalisierten Derivaten, muß gegebenenfalls auch eine entsprechende Schutzgruppentechnik verwendet werden. Ausgehend von den geeignet funktionalisierten monocyclischen Allylsulfiden, lassen sich dann die n-funktionellen cyclischen Allylsulfide der allgemeinen Formel I durch bekannte Bindungsknüpfungsreaktionen, wie Kondensations- (z.B. Ether-, Ester oder Amidbildung) oder Additionsreaktionen (z.B. Urethanbildung) mit entsprechend geeigneten n-funktionellen Precusorverbindungen herstellen:

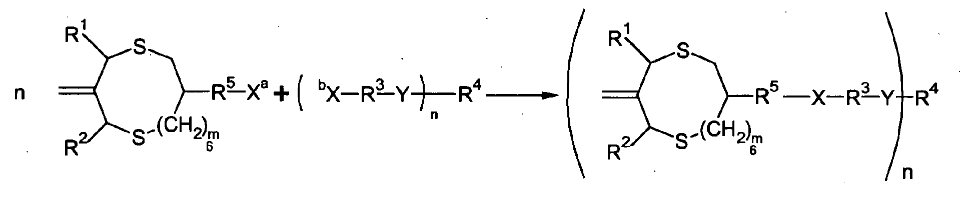

Konkretes Beispiel für n = 3:

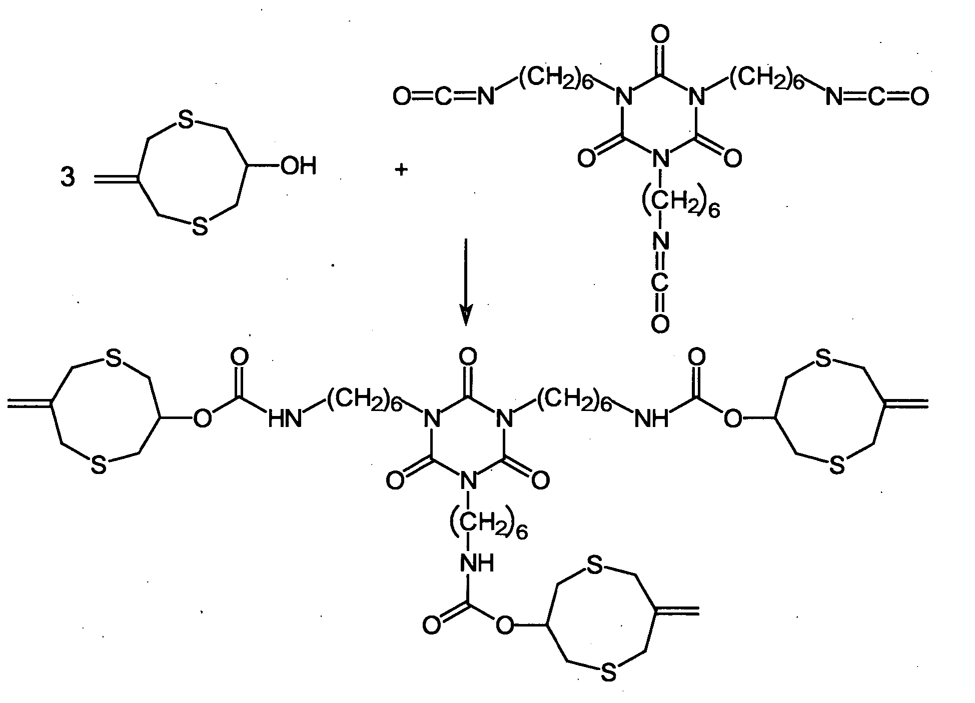

Bevorzugte cyclische Allylsulfid-Monomere der Formel (I) sind nachfolgend aufgeführt:

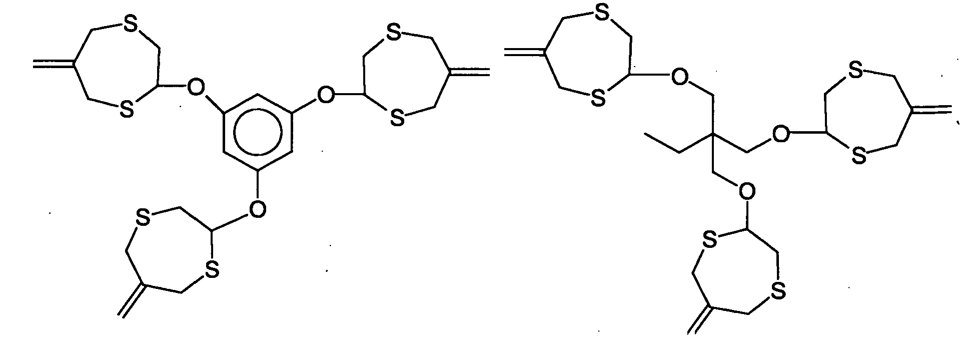

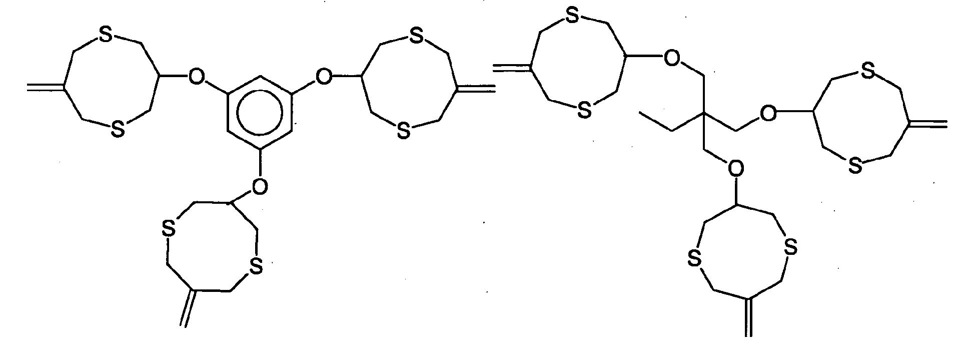

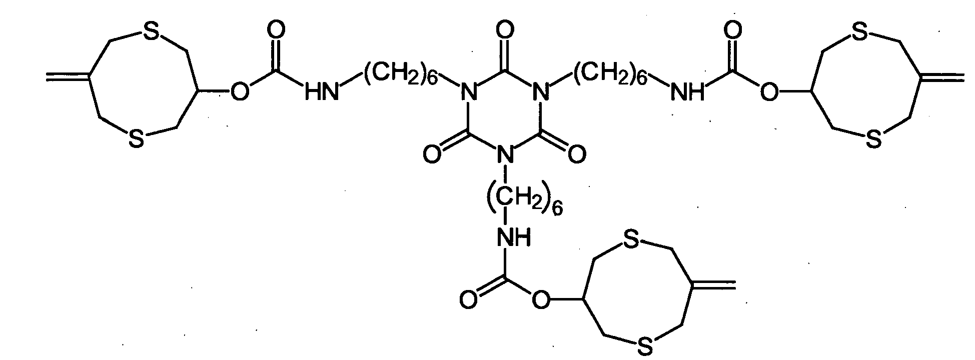

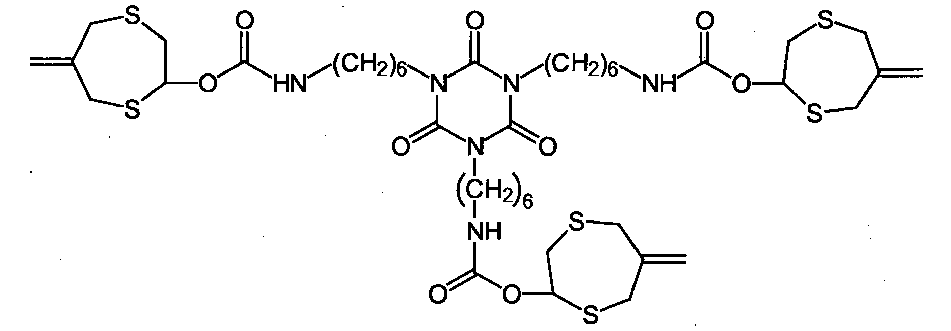

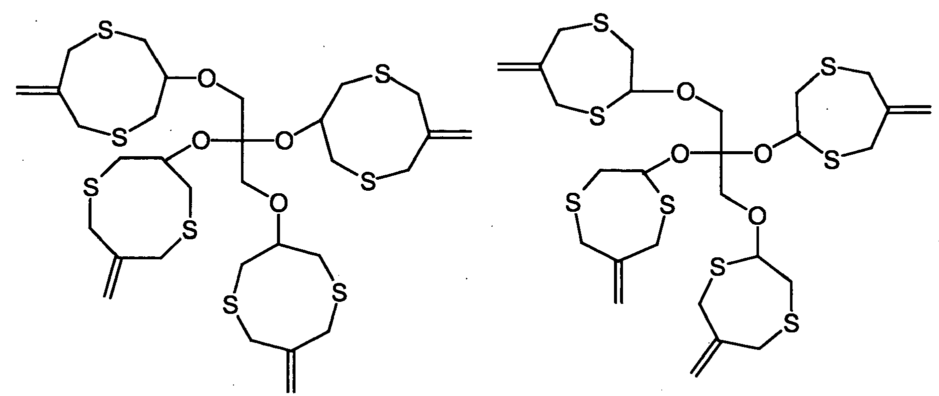

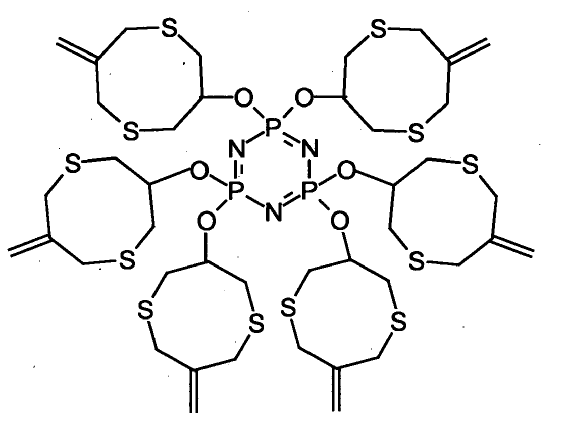

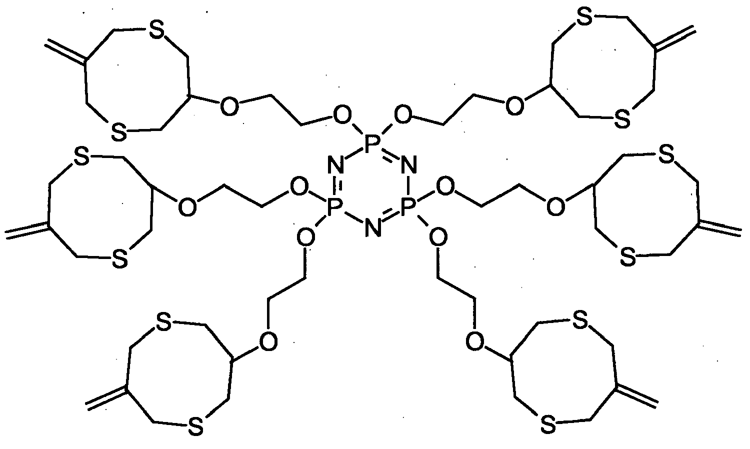

Die erfindungsgemäßen Dentalmaterialien auf der Basis der cyclischen Allylsulfide der Formel (I) lassen sich mit den bekannten radikalischen Initiatoren (vgl. Encylopedia of Polymer Science and Engineering, Vol. 13, Wiley-Intersci. Pub., New York etc. 1988, 754ff.) unter Ringöffnung polymerisieren.

Es eignen sich besonders Photoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich, wie Benzoinether, Dialkylbenzilketale, Dialkoxyacetophenone, Acylder Bisacylphosphinoxide, α-Diketone wie 9,10-Phenanthrenchinon, Diacetyl, Furil, Anisil, 4,4'-Dichlorbenzil und 4,4'-Dialkoxybenzil und Campherchinon.

Weiterhin können auch Azoverbindungen, wie 2,2'-Azobis(isobutyronitril) (AIBN) oder Azobis-(4-cyanovaleriansäure), oder Peroxide, wie Dibenzoylperoxid, Dilauroylperoxid, tert.-Butylperoctoat, tert.-Butylperbenzoat oder Di-(tert.-butyl)-peroxid verwendet werden. Als Initiatoren für die Heißhärtung eignen sich besonders auch Benzpinakol und 2,2'-Dialkylbenzpinakole.

Zur Beschleunigung der Initiierung von Peroxiden oder α-Diketonen werden vorzugsweise Kombinationen mit aromatischen Aminen eingesetzt. Bevorzugte Redoxsysteme sind: Kombinationen von Benzoylperoxid, Lauroylperoxid oder Campherchinon mit Aminen, wie insbesondere N,N-Dimethyl-p-toluidin, N,N-Dihydroxyethyl-p-toluidin, p-Dimethylaminobenzoesäureethylester oder strukturverwandte Systeme. Darüber hinaus sind auch Redoxsysteme geeignet, die Peroxide in Kombination mit Reduktionsmitteln, wie z.B. Ascorbinsäure, Barbituraten oder Sulfinsäuren enthalten.

Die erfindungsgemäßen Dentalmaterialien können als polymerisierbare Komponente ausschließlich cyclische Allylsulfide der Formel (I) oder eine Mischung derselben mit herkömmlichen radikalisch polymerisierbaren Monomeren, insbesondere mit monofunktionellen oder mehrfach funktionellen (Meth)acrylaten enthalten.

Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 (Meth)acrylgruppen verstanden. Mehrfach funktionelle Monomere haben vernetzende Eigenschaften.

Bevorzugte monofunktionelle (Meth)acrylate sind kommerziell verfügbare monofunktionelle Monomere, wie Methyl-, Ethyl-, Butyl-, Benzyl-, Furfuryl- oder Phenyl(meth)acrylat sowie 2-Hydroxyethyl- oder -propyl(meth)acrylat.

Besonders bevorzugt sind hydrolysestabile Monomere wie hydrolysestabile Mono(meth)acrylate, z.B. Mesitylmethacrylat oder 2-(Alkoyxymethyl)acrylsäuren, z.B. 2-(Ethoxymethyl)acrylsäure, 2-(Hydroxymethyl)acrylsäure, N-mono- oder -disubstitiuierte Acrylamide, wie z.B. N-Ethylacrylamid, N,N-Dimethacrylamid, N-(2-Hydroxyethyl)acrylamid oder N-Methyl-N-(2-hydroxyethyl)-acrylamid, und N-monosubstituierte Methacrylamide, wie z.B. N-Ethylmethacrylamid oder N-(2-Hydroxyethyl)methacrylamid sowie außerdem N-Vinylpyrrolidon und Allylether. Diese Monomere sind bei Raumtemperatur flüssig und eignen sich daher auch als Verdünner.

Bevorzugte mehrfach funktionelle Monomere sind Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, sowie Butandioldi(meth)-acrylat, 1,10-Decandioldi(meth)acrylat oder 1,12-Dodecandiol-di(meth)acrylat.

Besonders bevorzugt sind hydrolysestabile Vernetzermonomere, wie z.B. Urethane aus 2-(Hydroxymethyl)acrylsäure und Diisocyanten, wie 2,2,4-Trimethylhexamethylendiisocyanat oder Isophorondiisocyanat, vernetzende Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, Bis-(meth)acrylamide, wie z.B. N,N'-Diethyl-1,3-bis-(acrylamido)-propan, 1,3-Bis-(methacrylamido)-propan, 1,4-Bis-(acrylamido)-butan oder 1,4-Bis-(acryloyl)-piperazin, die durch Umsetzung aus den entsprechenden Diaminen mit (Meth)acrylsäurechorid synthetisiert werden können.

Die erfindungsgemäßen Dentalmaterialien auf der Basis der cyclischen Allylsulfide der Formel (I) sind säurestabil und können daher gemäß einer bevorzugten Ausführungsform mindestens ein radikalisch polymerisierbares, säuregruppenhaltiges Monomer enthalten, das in der Lage ist, die Zahnhartsubstanz anzuätzen, so daß eine Präkonditionierung der Zahnhartsubstanz mit Säure nicht notwendig ist. Säuregruppenhaltige Monomere werden im folgenden auch als acide Monomere bezeichnet. Solche säuregruppenhaltige Monomere verbessern die Haftung der Werkstoffe auf der Zahnhartsubstanz und machen so schrumpfungsarme Dentalmaterialien, wie z.B. Füllungskomposite oder Zemente, mit selbsthaftenden Eigenschaften zugänglich.

Bevorzugte Säuregruppen sind Carbonsäuregruppen, Phosphonsäuregruppen, Phosphatgruppen und/oder Sulfonsäuregruppen, wobei Gruppen mit mehr als einem aciden Wasserstoffatom teilweise verestert sein können. Besonders bevorzugt sind Monomere mit Phosphonsäuregruppen oder Phosphatgruppen. Die Monomere können eine oder mehrere acide Gruppen aufweisen, bevorzugte sind Verbindungen mit 1 bis 2 aciden Gruppen.

Bevorzugte polymerisationsfähige Carbonsäuren sind Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure.

Bevorzugte Phosphonsäuremonomere sind Alkenphosphonsäuren, Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methylpentyl-phosphonsäure, 2-[4-(Dihydroxyphosphoryl)-2-oxa-butyl]-acrylsäure und 2-[2-Dihydroxyphosphoryl)-ethoxymethyl]-acrylsäure-2,4,6-trimethyl-phenylester.

Bevorzugte acide polymerisationsfähige Phosphorsäureester (Phosphate) sind 2-Methacryloyloxypropylmono- und -dihydrogenphosphat, 2-Methacryloyloxyethylmono- und -dihydrogenphosphat, 2-Methacryloyloxyethyl-phenyl-hydrogenphosphat, Dipentaerythritol-pentamethacryloyloxyphosphat, 10-Methacryloyloxydecyl-dihydrogenphosphat, Dipentaerythritolpentamethacryloyloxyphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat.

Bevorzugte polymerisationsfähige Sulfonsäuren sind Vinylsulfonsäure, 4-Vinylphenylsulfonsäure oder 3-(Methacrylamido)propylsulfonsäure.

Weiterhin können die Dentalwerkstoffe zur Verbesserung der mechanischen Eigenschaften oder zur Einstellung der Viskosität organische oder anorganische, partikuläre oder faserförmige Füllstoffe enthalten. Bevorzugte anorganische partikuläre Füllstoffe sind amorphe kugelförmige Materialien, vorzugsweise mit einer Primärpartikelgröße von 10 bis 500 nm, auf der Basis von Oxiden, wie ZrO₂ und TiO₂ oder Mischoxiden aus SiO₂, ZrO₂ und/oder TiO₂, nanopartikuläre (Primärpartikelgröße von 10 bis 100 nm) oder mikrofeine Füllstoffe (mittlere Teilchengröße von 100 nm bis 5 µm), wie pyrogene Kieselsäure oder Fällungskieselsäure, sowie Minifüllstoffe, wie Quarz-, Glaskeramik- oder Glaspulver, vorzugsweise mit einer mittleren Teilchengröße von 0,1 bis 10 µm, sowie röntgenopake Füllstoffe, wie Ytterbiumtrifluorid, vorzugsweise mit einer mittleren Teilchengröße von etwa 200 nm, oder nanopartikuläres Tantal(V)-oxid oder Bariumsulfat, vorzugsweise mit einer Primärpartikelgröße von 10 bis 100 nm. Darüber hinaus können auch Glasfasern, Polyamid- oder Kohlenstoff-Fasern eingesetzt werden.

Außerdem können die erfindungsgemäßen Dentalwerkstoffe ein oder mehrere weitere Additive enthalten, wie Stabilisatoren, UV-Absorbern, Farbstoffen, Pigmenten und/oder Gleitmittel. Im Falle von Adhäsiven können auch Lösungsmittel wie Ethanol, Aceton oder deren Mischungen mit Wasser eingesetzt werden.

Die cyclischen Allylsulfide der Formel (I) eignen sich besonders zur Herstellung von dentalen Füllungskompositen, Befestigungszementen, Adhäsiven, Beschichtungsmaterialien und Formkörpern. Außerdem eignen sie sich zur Herstellung von Werkstoffen für Inlays/Onlays, künstliche Zähne und Verblendmaterialien für Kronen und Brücken.

Ein weiterer Gegenstand der Erfindung ist ein Verfahren zur Herstellung von gehärteten Formkörpern. Hierzu wird ein erfindungsgemäßer Dentalwerkstoff zu einem Formkörper, vorzugsweise einer Krone, Brücke, einem Inlay oder Onlay, einem künstlichen Zahn oder anderen Dentalrestauration geformt und anschließend zumindest teilweise gehärtet. Das Formen geschieht auf an sich bekannte Weisen.

Die erfindungsgemäßen Dentalwerkstoffe zeichnen sich durch einen geringeren Polymerisationschrumpf, sehr gute mechanische Eigenschaften und bei Verwendung säuregruppenhaltiger Monomere durch eine hohe Eigenhaftung auf der Zahnhartsubstanz aus.

Die erfindungsgemäßen Dentalwerkstoffe enthalten vorzugsweise:

| | |
|---|---|
| 1 bis 95 Gew.-% | Allylsulfid gemäß Formel (I); |
| 0,01 bis 5 Gew.-% | Initiator für die radikalische Polymerisation; |
| 0 bis 60 Gew.-% | weiteres radikalisch polymerisierbares Monomer; |
| 0 bis 2 0 Gew.-% | säuregruppenhaltiges Monomer; |
| 0 bis 85 Gew.-% | Füllstoff. |

Soweit nicht anders angegeben beziehen sich alle Gew.-%-Angaben auf die Gesamtmasse des Werkstoffs. Die weiteren radikalisch polymerisierbaren Monomere schließen nicht die säuregruppenhaltigen Monomere ein.

Die genaue Zusammensetzung richtet sich dabei nach dem gewünschten Anwendungszweck. Dentalwerkstoffe zur Verwendung als Füllungskomposite enthalten vorzugsweise:
1 bis 45 Gew.-%, besonders bevorzugt 10 bis 30 Gew.-% cyclisches Allylsulfid gemäß Formel (I),
0,01 bis 5 Gew.-%, besonders bevorzugt 0,1 bis 2,0 Gew.-% Initiator für die radikalische Polymerisation,
0 bis 50 Gew.-%, besonders bevorzugt 0 bis 10 Gew.-% zusätzliches radikalisch polymerisierbares Monomer,
30 bis 85 Gew.-%, besonders bevorzugt 40 bis 80 Gew.-% Füllstoff,
0 bis 10 Gew.-% säuregruppenhaltiges Monomer.

Dentalwerkstoffe zur Verwendung als Zemente enthalten vorzugsweise:
1 bis 60 Gew.-%, besonders bevorzugt 20 bis 50 Gew.-% cyclisches Allylsulfid der Formel (I),
0,01 bis 5 Gew.-%, besonders bevorzugt 0,1 bis 2,0 Gew.-% Initiator für die radikalische Polymerisation,
0 bis 60 Gew.-%, besonders bevorzugt 0 bis 20 Gew.-% zusätzliches radikalisch polymerisierbares Monomer,
20 bis 60 Gew.-%, besonders bevorzugt 30 bis 60 Gew.-% Füllstoff,
0 bis 15 Gew.-% säuregruppenhaltiges Monomer.

Dentalwerkstoffe zur Verwendung als Beschichtungsmaterialien enthalten vorzugsweise:
1 bis 95 Gew.-%, besonders bevorzugt 10 bis 60 Gew.-% cyclisches Allylsulfid gemäß Formel (I),
0,01 bis 5 Gew.-%, besonders bevorzugt 0,1 bis 2,0 Gew.-% Initiator für die radikalische Polymerisation,
0 bis 60 Gew.-%, besonders bevorzugt 0 bis 40 Gew.-% zusätzliches radikalisch polymerisierbares Monomer,
0 bis 20 Gew.-% Füllstoff,
0 bis 10 Gew.-% säuregruppenhaltiges Monomer.

Dentalwerkstoffe zur Verwendung als Adhäsive enthalten vorzugsweise:
1 bis 80 Gew.-%, besonders bevorzugt 10 bis 60 Gew.-% cyclisches Allylsulfid gemäß Formel (I),
0,01 bis 5 Gew.-%, besonders bevorzugt 0,1 bis 2,0 Gew.-% Initiator für die radikalische Polymerisation,
0 bis 60 Gew.-%, besonders bevorzugt 0 bis 40 Gew.-% zusätzliches radikalisch polymerisierbares Monomer,
0 bis 40 Gew.-%, besonders bevorzugt 0 bis 30 Gew.-% Lösungsmittel,
0 bis 20 Gew.-% Füllstoff,
0 bis 20 Gew.-% säuregruppenhaltiges Monomer.

Die Erfindung wird im Folgenden anhand von Beispielen näher erläutert.

### Ausführungsbeispiele

### Beispiel 1: Synthese des Additionsproduktes des Trimers von Hexamethylendiisocyanat mit 7-Hydroxy-3-methylen-1,5-dithiacylooctan (s-TDTO)

50,5 g (0,1 mol) symmetrisches Hexamethylendiisocynat-Trimer (Desmodur N 3300, Bayer AG; Scherviskosität bei 40 °C = 4,3 Pas) wurden bei Raumtemperatur zu einer Lösung von 52,9 g (0,3 mol) 7-Hydroxy-3-methylen-1,5-dithiacylooctan, das durch Veretherung von 2-Hydroxy-1,3-dichlorpropan mit 3-Mercapto-2-mercaptomethyl-1-propen zugänglich ist, 12 mg TEMPO (2,2,6,6-Tetramethyl-piperidin-1-oxyl, Inhibitor), 25 mg MEHQ (Hydrochinonmonomethylether, Stabilisator) und 0,2 g Metatin 812 (Dibutylzinndioctoat, Katalysator) in 100 ml Methylenchlorid getropft. Nach 48 h Rühren war im IR-Spektrum keine Isocyanatbande mehr zu erkennen. Das klare Reaktionsgemisch wurde zweimal mit je 100 ml 1,0 N NaOH und dreimal mit je 100 ml gesättigter Kochsalzlösung gewaschen. Dann wurde die organische Phase mit Natriumsulfat getrocknet, mit 20 mg MEHQ stabilisiert, und das Lösungsmittel am Rotationsverdampfer unter Lufteinleiten vollständig entfernt. Es ergaben sich 95,5 g (Ausbeute: 92 %) eines weissen Feststoffes des trifunktionellen Monomeren **s-TDTO** mit einem Schmelzpunkt von 55,1 °C und der Struktur: 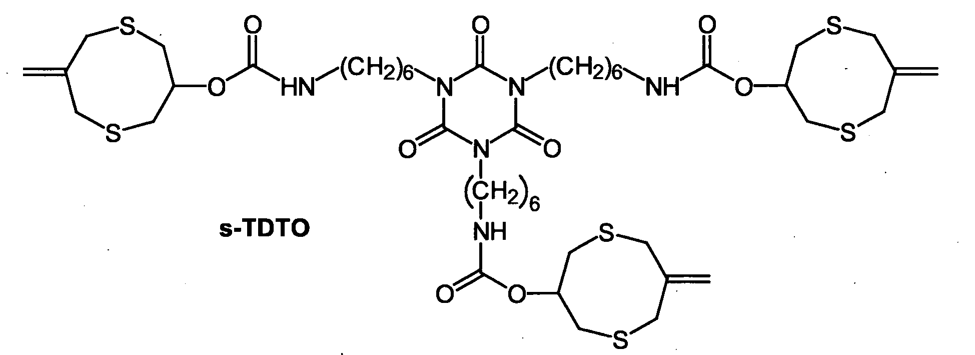 ¹H-NMR (CDCl₃): 1,27 (m, 12 H, CH₂), 1,48 (m, 6H, CH₂), 1,63 (br, 6H, CH₂), 3,02 (m, 12H, S-CH₂-CH-CH₂-S) 3,12 (m, 6H, CH₂N), 3,24 (m, 12H, =CH₂S), 3,86 (t, 6H, CH₂N), 4,92 (br, m, 6H, CH und NH), 5,23 (s, 6H, =CH₂) ppm.

### Beispiel 2: Synthese des Additionsproduktes des asymmetrischen Trimers von Hexamethylendiisocyanat mit 7-Hydroxy-3-methylen-1,5-dithiacylooctan (a-TDTO)

Analog zu Beispiel 1 wurden 50,5 g (0,1 mol) asymmetrisches Hexamethylendiisocyanat-Trimer (Desmodur VP LS 2294, Bayer AG; Scherviskosität bei 40 °C = 1,3 Pas) mit 52,9 g (0,3 mol) 7-Hydroxy-3-methylen-1,5-dithiacylooctan umgesetzt und aufgearbeitet. Es ergaben sich 92 g (Ausbeute: 89 %) des trifunktionellen Monomer **a-TDTO** als farblose hochviskose Flüssigkeit mit nachfolgender Struktur:

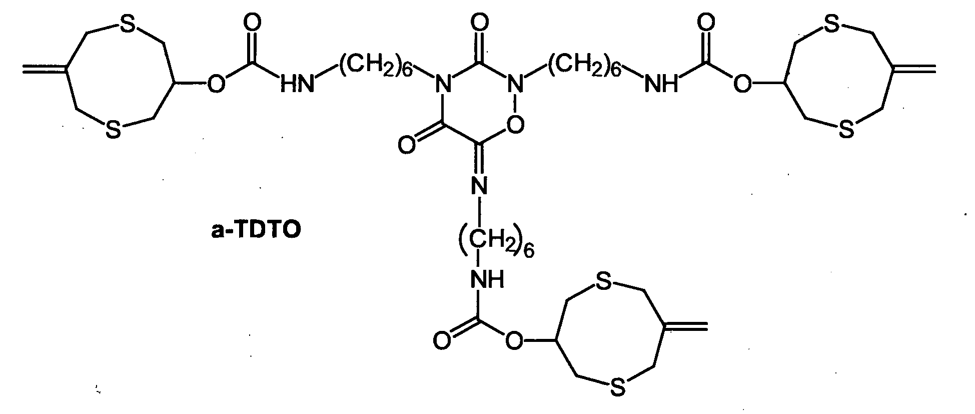

### Beispiel 3: Herstellung eines Dentalzementes auf der Basis des cyclischen Allylsulfids aus Beispiel 2

Entsprechend der nachfolgend aufgeführten Tabelle 1 wurden Kompositbefestigungszemente auf der Basis von (A) einer herkömmlichen Methacrylatmischung (Vergleich) und (B) des trifunktionellen cyclischen Allylsulfids **a-TDTO** aus Beispiel 2 mittels eines Walzenstuhles (Typ Exakt, Exakt Apparatebau, Norderstedt) hergestellt. Von den Materialien wurden Prüfkörper präpariert, die zweimal für jeweils 3 Minuten mit einer dentalen Lichtquelle (Spectramat^{®}, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit, des Biege-E-Moduls und der Exothermzeit.

Aus Tabelle 2 ist ersichtlich, daß das Material B mechanische Eigenschaften aufweist, die mit denen des Materials A auf der Basis einer rein konventionellen Methacrylatmischung vergleichbar sind, und daß die Reaktivität des Materials B (Exothermzeit nur 8 s) sogar höher als die des Vergleichsmaterials ist (Exothermzeit 13 s).

**Tabelle 1: Zusammensetzung der Zemente**

| **Bestandteil** | **Material A^{*)} Anteile (Gew.-%)** | **Material B Anteile (Gew.-%)** |
|---|---|---|
| Urethandimethacrylat¹⁾ | 31,6 | 31,6 |
| Decandioldimethacrylat | 7,8 | - |
| a-TDTO(Bsp. 2) | - | 7,8 |
| Aerosil OX-50 (Degussa) | 41,2 | 41,2 |
| Ytterbiumtrifluorid (Rhone-Poulenc) | 18,7 | 18,7 |
| Photoinitiator²⁾ | 0,7 | 0,7 |

| | | |
|---|---|---|
| ^{*)} Vergleichsmaterial ¹⁾ Urethandimethacrylat aus 2 mol 2-Hydroxyethylmethacrylat und 1 mol 2,2,4-Trimethylhexamethylendiisocyanat-1,6 ²⁾ Mischung aus Campherchinon (0,17 %), p-N,N-Dimethylaminobenzoesäureethylester (0,30 %), Lucirin TPO (0,23 %, BASF) | | |

**Tabelle 2: Materialeigenschaften der Zemente**

| **Materialeigenschaft** | **Material A^{*)}** | **Material B** |
|---|---|---|
| Biegefestigkeit (MPa) nach 24 h | 120 | 120 |
| Biegefestigkeit (MPa) nach 24 h WL¹⁾ | 120 | 125 |
| Biegefestigkeit (MPa) nach 7 d WL | 123 | 129 |
| Biege-E-Modul (GPa) nach 24 h | 6,39 | 5,78 |
| Biege-E-Modul (GPa) nach 24 h WL | 6,32 | 6,45 |
| Biege-E-Modul (GPa) nach 7 d WL | 6,21 | 6,05 |
| Exothermzeit(s) | 13 | 8 |

| | | |
|---|---|---|
| ^{*)} Vergleichsmaterial ¹⁾ WL = Wasserlagerung der Prüfkörper | | |

### Beispiel 4: Herstellung eines Füllungskomposits auf der Basis des cyclischen Allylsulfids aus Beispiel 2

Entsprechend der nachfolgend aufgeführten Tabelle 3 wurde ein Füllungskomposit auf der Basis einer Methacrylatmischung unter Einbeziehung des Monomer **a-TDTO** aus Beispiel 2 mittels eines Laborkneters (Type LPM 0.5 SP, Fa. Linden, Marienheide) hergestellt. Von den Materialien wurden Prüfkörper präpariert, die zweimal für jeweils 3 Minuten mit einer dentalen Lichtquelle (Spectramat^{®}, Ivoclar Vivadent AG) bestrahlt und damit ausgehärtet wurden. Nach der ISO-Norm ISO-4049 (Dentistry - Polymer-based filling, restorative and luting materials) wurde die Biegefestigkeit, der Biege-E-Modul und der Polymerisationsschrumpf bestimmt.

**Tabelle 3: Zusammensetzung des Komposits**

| **Bestandteil** | **Anteile (Gew.-%)** |
|---|---|
| a-TDTO (Bs p. 2) | 19,74 |
| SR-348C (Satomer)¹⁾ | 6,90 |
| Glasfüller GM27884²⁾ | 72,00 |
| Photoinitiator³⁾ | 0,36 |

| | |
|---|---|
| ¹⁾ ethoxyliertes Bisphenol-Dimethacrylat, das hauptsächlich 3 Oxyethylengruppen enthält ²⁾ Bariumaluminiumsilikatglas (Schott) silanisiert, mittlere Partikelgrösse 1,0 µm ³⁾ Mischung aus Campherchinon (0,06 %), p-N,N-Dimethylaminobenzoesäureethylester (0,12 %), Lucirin TPO (0,08 %, BASF) und Diphenyliodoniumhexafluorophosphat (0,10 %) | |

**Tabelle 4: Materialeigenschaften des Komposits**

| **Materialeigenschaft** | **Meßwert** |
|---|---|
| Biegefestigkeit (MPa) nach 24 h WL¹⁾ | 131 |
| Biege-E-Modul (GPa) nach 24 h WL¹⁾ | 9,92 |
| Polymerisationsschrumpf (%) nach 24 h | 1,71 |

| | |
|---|---|
| ¹⁾ WL = Wasserlagerung der Prüfkörper bei 37 °C | |

Das Beispiel zeigt, daß mit dem cyclischen Allylsulfid **a-TDTO** aus Beispiel 2 Füllungskomposite mit guten mechanischen Eigenschaften zugänglich sind. Der dilatometrisch ermittelte Polymerisationsschrumpf betrug nur 1,7 % im Vergleich zu 2,7 % von einem Vergleichskomposit bei dem eines reines Dimethacrylat-Harz als Matrix verwendet wurde.

### Beispiel 5: Herstellung eines Füllungskomposits auf der Basis des cyclischen Allylsulfids aus Beispiel 2 und des aciden Monomers MDP

Entsprechend der nachfolgend aufgeführten Tabelle 5 wurde ein Füllungskomposit auf der Basis einer Methacrylatmischung unter Einbeziehung des Monomers **a-TDTO** aus Beispiel 2 und des aciden Monomeren **MDP** (= 10-(Methacryloyloxy)-decyldihydrogenphosphat) mittels eines Laborkneters (Typ LPM 0.5 SP, Fa. Linden, Marienheide) hergestellt.

**Tabelle 5: Zusammensetzung des Komposits**

| **Stoffe** | **Anteile (Gew.-%)** |
|---|---|
| a-TDTO (B s p . 2) | 17,34 |
| SR-348C (Satomer) | 5,70 |
| MDP | 3,60 |
| Glasfüller GM27884¹⁾ | 72,00 |
| Photoinitiator²⁾ | 0,36 |

| | |
|---|---|
| ¹⁾ Bariumaluminiumsilikatglas (Schott) silanisiert, mittlere Partikelgrösse 1,0 µm ²⁾ Mischung aus Campherchinon (0,06 %), p-N,N-Dimethylaminobenzoesäureethylester (0,12 %), Lucirin TPO (0,08 %, BASF) und Diphenyliodoniumhexafluorophosphat (0,10 %) | |

Von dem Komposit wurde die Dentinhaftung gemäß ISO/TS 11405 (Dental materials - Testing of adhesion to tooth structure) bestimmt. Dabei wurde wie folgt vorgegangen: Von kariesfreien Rinder-Zähnen wird die Pulpa und die Wurzel entfernt. Die Zähne werden in einem Epoxidharz eingebettet und in Wasser bei 37 °C gelagert. Vor dem Gebrauch wurden die Zähne mit Schleifpapier (600 grid) angeschliffen, bis eine ebene Dentinoberfläche sichtbar wird. Diese Fläche wurde mit Wasser gewaschen und leicht angetrocknet. Der eingebettete Zahn wurde dann in einer geeigneten Halterung fixiert. Ein Ring mit einem Innendurchmesser von 4 mm und einer Höhe von 4 mm wurde auf der Zahnoberfläche fixiert. Das Komposit wurde in einer Schichtstärke von ca. 2 mm in den Ring gestopft und mit einer Dentallampe (Bluephase) 20 Sekunden ausgehärtet. Eine zweite Schicht Komposit wurde über der ersten Schicht appliziert und ebenfalls ausgehärtet. Der Ring wurde entfernt und der Zahn-Komposit-Verbund in Wasser bei 37 °C gelagert. Nach 24 Stunden wurde das aufpolymerisierte Komposit mit einer in der ISO/TS 11405 beschriebenen Apparatur abgeschert. Der Kraftaufwand, der hierzu erforderlich war, wurde durch die Querschnittsfläche des Prüfkörpers dividiert, wodurch sich für das getestete Komposit eine Scherfestigkeit von 8,6 N/mm² ergab. Diese Dentinhaftung stellt für ein selbsthaftendes Komposit einen sehr guten Wert dar.

## Patentansprüche

1. Dentalwerkstoff, der mindestens ein multicyclisches Allylsulfid mit der allgemeinen Formel (I) enthält: 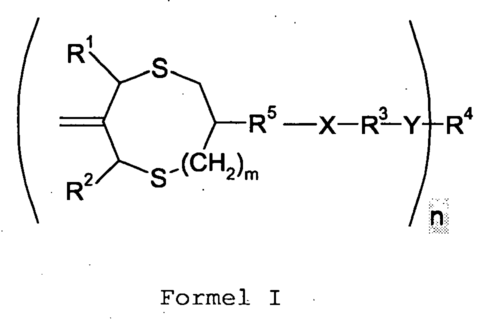 in der R¹ bis R⁴, X, Y, m und n unabhängig voneinander die folgenden Bedeutungen haben:
R¹ = H oder ein C₁-C₁₀-Alkyl-Rest,
R² = H oder ein C₁-C₁₀-Alkyl-Rest,
R³ = entfällt oder ein C₁-C₂₀-Alkylen-Rest, der durch O oder S unterbrochen sein kann, ein cycloaliphatischer C₄-C₁₂-Rest, ein bicyclischer C₆-C₁₂-Rest, ein C₆-C₁₄-Arylen- oder C₇-C₂₀-Alkylenarylen-Rest,
R⁴ = ein n-fach substituierter aliphatischer C₂- bis C₂₀-Kohlenwasserstoffrest, der durch O oder S unterbrochen sein kann, ein cycloaliphatischer C₄-C₁₂-Rest, ein aromatischer C₆-C₁₄-Rest, ein aliphatischaromatischer C₇-C₂₀-Rest oder ein heterocyclischer Rest, der 4 bis 20 Kohlenstoffatome und 1 bis 6 Heteroatome enthalten kann, die aus N-, O-, P- und/oder S-Atomen ausgewählt sind, oder der ausschließlich durch diese Heteroatome gebildet wird,
R⁵ = entfällt oder ein C₁-C₁₀-Alkylen-Rest,
X = entfällt, O, S, -O-CO- oder -O-CO-NH-,
Y = entfällt, O, S, -O-CO- oder -O-CO-NH-,
m = 0 oder 1 und
n = eine ganze Zahl von 3 bis 6.

2. Dentalwerkstoff nach Anspruch 1, bei dem mindestens eine der Variablen eine der folgenden Bedeutungen hat:
R¹ = H oder Methyl,
R² = H oder Methyl,
R³ = entfällt, ein C₁-C₆-Alkylen-Rest, ein cycloaliphatischer C₄-C₆-Rest oder ein C₆-C₁₂-Arylen-Rest,
R⁴ = ein n-fach substituierter aliphatischer C₂- bis C₁₀-Rest, ein cyclo-aliphatischer C₄-C₅-Rest, ein aromatischer C₆-C₁₂-Rest, ein heterocyclischer C₄-C₁₂-Rest, der 1 bis 6 Heteroatome enthalten kann, einen gesättigten oder ungesättigten Rest einer Phosphor-Stickstoffverbindung,
R5 = entfällt oder ein C₁-C₃-Alkylen-Rest,
X = entfällt, O, -O-CO- oder -O-CO-NH-,
Y = entfällt, O, -O-CO- oder -O-CO-NH-,
m = 0 oder 1 und
n = 3 oder 4.

3. Dentalwerkstoff nach Anspruch 2, bei dem mindestens eine der Variablen eine der folgenden Bedeutungen hat:
R¹ = H oder Methyl,
R² = H oder Methyl,
R³ = entfällt oder ein C₁₋C₆-Alkylen-Rest,
R⁴ = ein n-fach substituierter aliphatischer C₂- bis C₆-Rest, aromatischer C₆-C₁₀-Rest, ein Cyanursäure- oder Isocyanursäure-Rest, der 1 bis 6 Heteroatome enthalten kann, ein cyclischer Triphosphazenrest,
R⁵ = entfällt,
X = O oder -O-CO-NH-,
Y = entfällt oder O,
m = 0 oder 1 und
n = 3 oder 4.

4. Dentalwerkstoff nach einem der vorhergehenden Ansprüche, der zusätzlich mindestens einen Initiator für die radikalische Polymerisation enthält.

5. Dentalwerkstoff nach einem der vorhergehenden Ansprüche, der zusätzlich mindestens ein weiteres radikalisch polymerisierbares Monomer enthält.

6. Dentalwerkstoff nach Anspruch 5, der ein radikalisch polymerisierbares Monomer mit 2 bis 3 radikalisch polymerisierbaren Gruppen enthält.

7. Dentalwerkstoff nach einem der vorhergehenden Ansprüche, der zusätzlich mindestens säuregruppenhaltiges radikalisch polymerisierbares Monomer enthält.

8. Dentalwerkstoff nach Anspruch 7, bei dem das säuregruppenhaltige Monomer als acide Gruppe eine Carbonsäuregruppe, Phosphonsäuregruppe, Phosphatgruppe und/oder Sulfonsäuregruppe enthält.

9. Dentalwerkstoff nach einem der vorhergehenden Ansprüche, der zusätzlich mindestens einen Füllstoff enthält.

10. Dentalwerkstoff nach einem der vorhergehenden Ansprüche, der
| | |
|---|---|
| 1 bis 95 Gew.-% | Allylsulfid gemäß Formel (I); |
| 0,01 bis 5 Gew.-% | Initiator für die radikalische Polymerisation; |
| 0 bis 60 Gew.-% | weiteres radikalisch polymerisierbares Monomer; |
| 0 bis 20 Gew.-% | säuregruppenhaltiges Monomer; |
| 0 bis 85 Gew.-% | Füllstoff enthält. |

11. Dentalwerkstoff nach Anspruch 10 zur Verwendung als Füllungskomposit, der
| | |
|---|---|
| | |
| 1 bis 45 Gew.-% | Allylsulfid gemäß Formel (I); |
| 0,01 bis 5 Gew.-% | Initiator für die radikalische Polymerisation; |
| 0 bis 50 Gew.-% | weiteres radikalisch polymerisierbares Monomer; |
| 30 bis 85 Gew.-% | Füllstoff; |
| 0 bis 10 Gew.-% | säuregruppenhaltiges Monomer enthält. |

12. Dentalwerkstoff nach Anspruch 10 zur Verwendung als Zement, der
| | |
|---|---|
| 1 bis 60 Gew.-% | Allylsulfid gemäß Formel (I); |
| 0,01 bis 5 Gew.-% | Initiator für die radikalische Polymerisation; |
| 0 bis 60 Gew.-% | weiteres radikalisch polymerisierbares Monomer; |
| 20 bis 60 Gew.-% | Füllstoff; |
| 0 bis 15 Gew.-% | säuregruppenhaltiges Monomer enthält. |

13. Dentalwerkstoff nach Anspruch 10 zur Verwendung als Beschichtungsmaterial, der
| | |
|---|---|
| 1 bis 95 Gew.-% | Allylsulfid gemäß Formel (I); |
| 0,01 bis 5 Gew.-% | Initiator für die radikalische Polymerisation; |
| 0 bis 60 Gew.-% | weiteres radikalisch polymerisierbares Monomer; |
| 0 bis 20 Gew.-% | Füllstoff; |
| 0 bis 10 Gew.-% | säuregruppenhaltiges Monomer enthält. |

14. Dentalwerkstoff nach Anspruch 10 zur Verwendung als Adhäsiv, der
| | |
|---|---|
| 1 bis 80 Gew.-% | Allylsulfid nach Formel (I); |
| 0,01 bis 5 Gew.-% | Initiator für die radikalische Polymerisation; |
| 0 bis 60 Gew.-% | weiteres radikalisch polymerisierbares Monomer; |
| 0 bis 20 Gew.-% | Füllstoff; |
| 0 bis 20 Gew.-% | säuregruppenhaltiges Monomer; |
| 0 bis 40 Gew.-% | Lösungsmittel enthält. |

15. Verwendung eines Allylsulfids gemäß Formel (I) zur Herstellung eines Dentalwerkstoffs.

16. Verwendung nach Anspruch 15 zur Herstellung eines Füllungskomposits, Zements, Beschichtungsmaterials oder Adhäsivs.

17. Verfahren zur Herstellung von gehärteten Formkörpern bei dem man einen Dentalwerkstoff gemäß einem der Ansprüche 1 bis 14 zu einem Formkörper formt und anschließend zumindest teilweise härtet.

18. Verfahren nach Anspruch 17, bei dem der Formkörper eine Krone, Brücke, ein Inlay, Onlay, ein künstlicher Zahn oder eine andere Dentalrestauration ist.

## Claims

1. A dental material comprising at least one multicyclic allyl sulphide with the general Formula (I): 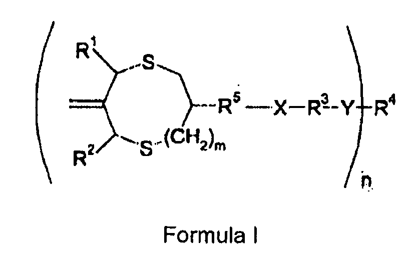 in which R¹ to R⁴, X, Y, m and n, independently of one another, have the following meanings:
R¹ = H or a C₁-C₁₀ alkyl group;
R² = H or a C₁-C₁₀ alkyl group;
R³= is absent or is a C₁-C₂₀ alkylene group that can be interrupted by O or S, a cycloaliphatic C₄-C₁₂ group, a bicyclic C₆-C₁₂ group, a C₆-C₁₄ arylene or C₇-C₂₀ alkylene arylene group;
R⁴ = an n-times substituted aliphatic C₂ to C₂₀ hydrocarbon group that can be interrupted by O or S, a cycloaliphatic C₄-C₁₂ group, an aromatic C₆-C₁₄ group, an aliphatic aromatic C₇-C₂₀ group or a heterocyclic group that can contain 4 to 20 carbon atoms and 1 to 6 heteroatoms that are selected from N, O, P or S atoms or which is formed exclusively by these heteroatoms;
R⁵ = is absent or is a C₁-C₁₀ alkylene group;
X = is absent or is O, S, -O-CO- or -O-CO-NH-;
Y = is absent or is O, S, .-O-CO- or -O-CO-NH-;
m = 0 or 1; and
n = an integer from 3 to 6

2. The dental material according to claim 1, in which at least one of the variables has one of the following meanings:
R¹ = H or methyl;
R² = H or methyl;
R³ = is absent or is a C₁-C₆ alkylene group, a cycloaliphatic C₄-C₆ group or a C₆-C₁₂ arylene group;
R⁴ = an n-times substituted aliphatic C₂ to C₁₀ group, a cycloaliphatic C₄-C₅ group, an aromatic C₆-C₁₂ group, a heterocyclic C₄-C₁₂ group that can contain 1 to 6 heteroatoms, a saturated or unsaturated group of a phosphorus nitrogen compound;
R⁶ = is absent or is a C₁-C₃ alkylene group;
X = is absent or is O, -O-CO- or -O-CO-NH;
Y = is absent or Is O, -O-CO- or -O-CO-NH-;
m = 0 or 1; and
n = 3 or 4.

3. The dental material according to claim 2, in which at least one of the variables has the following meaning:
R¹ = H or methyl;
R² = H or methyl;
R³ = is absent or is a C₁-C₆ alkylene group;
R⁴ = an n-times substituted aliphatic C₂ to C₆ group, aromatic C₆-C₁₀ group, a cyanuric acid or isocyanuric acid group which can contain 1 to 6 heteroatoms, a cyclic triphosphazene group;
R⁵ = is absent;
X = O or -O-CO-NHµ-;
Y = is absent or is O;
m = 0 or 1; and
n = 3 or 4.

4. Dental material according to one of the preceding claims, further comprising at least one initiator for the radical polymerisation .

5. Dental material according to one of the preceding claims, further comprising at least one radically polymerisable monomer.

6. Dental material according to claim 5, comprising a radically polymerisable monomer containing 2 to 3 radically polymerisable groups.

7. Dental material according to one of the preceding claims, further comprising at least one acid group-containing, radically polymerisable monomer

8. Dental material according to claim 7, wherein the acid group-containing monomer comprises a carboxylic acid group, phosphonic acid group, phosphate group and/or sulfonic acid group as the acidic group.

9. Dental material according to one of the preceding claims, further comprising at least one filler.

10. Dental material according to one of the preceding claims, comprising:
| | |
|---|---|
| 1 to 95 wt.% | allyl sulphide according to Formula (I); |
| 0 01 to 5 wt % | initiator for the radical polymerisation; |
| 0 to 60 wt % | additional radically polymerisable monomer; |
| 0 to 20 wt.% | acid group-containing monomer; |
| 0 to 85 wt.% | filler |

11. Dental material according to claim 10 for use as a filling composite, comprising:
| | |
|---|---|
| 1 to 45 wt,% | allyl sulphide according to Formula (I); |
| 0.01 to 5 wt.% | initiator for the radical polymerisation; |
| 0 to 50 wt.% 30 to 85 wt.% | additional radically polymerisable monomer; filler; |
| 0 to 10 wt % | acid group containing monomer. |

12. Dental material according to claim 10 for use as cement, comprising:
| | |
|---|---|
| 1 to 60 wt % | allyl sulphide according to Formula (I); |
| 0.01 to 5 wt % | initiator for the radical polymerisation; |
| 0 to 60 wt.% | additional radically polymerisable monomer; |
| 20 to 60 wt % | filler; |
| 0 to 15 wt.% | acid group-containing monomer |

13. Dental material according to claim 10 for use as coating material, comprising:
| | |
|---|---|
| 1 to 95 wt.% | allyl sulphide according to Formula (I); |
| 0.01 to 5 wt.% | initiator for the radical polymerisation; |
| 0 to 60 wt.% | additional radically polymerisable monomer; |
| 0 to 20 wt % | filler; |
| 0 to 10 wt.% | acid group-containing monomer |

14. Dental material according to claim 10 for use as an adhesive, comprising:
| | |
|---|---|
| 1 to 80 wt % | allyl sulphide according to Formula (I); |
| 0.01 to 5 wt % | initiator for the radical polymerisation; |
| 0 to 60 wt.% | additional radically polymerisable monomer; |
| 0 to 20 wt % | filler; |
| 0 to 20 wt.% | acid group-containing monomer. |

15. Use of an allyl sulfide according to Formula (I) for manufacturing a dental material.

16. Use according to claim 15 for manufacturing a filling composite, cement, coating material or adhesive.

17. Process for manufacturing cured moulded articles, wherein a dental material according to one of claims 1 to 14 is moulded to form a moulded article and subsequently at least partially cured

18. Process according to claim 17, wherein the moulded article is a crown, bridge, an inlay, onlay, an artificial tooth or another dental restoration.

## Revendications

1. Matériau dentaire, qui contient au moins un sulfure d'allyle multicyclique de formule générale (I) 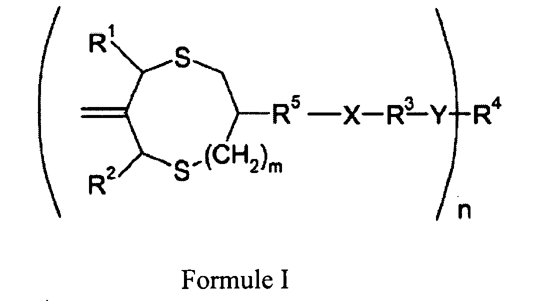 dans laquelle R¹ à R⁴, X, Y, m et n ont, indépendamment les uns des autres, les significations suivantes :
R¹ représente un atome d'hydrogène ou un radical alkyle en C₁-C₁₀,
R² représente un atome d'hydrogène ou un radical alkyle en C₁-C₁₀,
R³ est absent ou représente un radical alkylène en C₁-C₂₀, qui peut être interrompu par O ou S, un radical cycloaliphatique en C₄-C₁₂, un radical bicyclique en C₆-C₁₂, un radical arylène en C₆-C₁₄ ou alkylène-arylène en C₇-C₂₀,
R⁴ représente un radical hydrocarboné aliphatique en C₂-C₂₀ n-fois substitué, qui peut être interrompu par O ou S, un radical cycloaliphatique en C₄-C₁₂, un radical aromatique en C₆-C₁₄, un radical aliphatique-aromatique en C₇-C₂₀ ou un radical hétérocyclique qui peut contenir de 4 à 20 atomes de carbone et de 1 à 6 hétéroatomes choisis parmi les atomes d'azote, d'oxygène, de phosphore et/ou de soufre, ou qui est exclusivement formé par ces hétéroatomes,
R⁵ est absent ou représente un radical alkylène en C₁-C₁₀,
X est absent ou représente O, S, -O-CO- ou -O-CO-NH-,
Y est absent ou représente O, S, -O-CO- ou -O-CO-NH-,
m vaut 0 ou 1 et
n représente un nombre entier valant de 3 à 6.

2. Matériau dentaire selon la revendication 1, dans lequel au moins l'une des variables a l'une des significations suivantes :
R¹ représente un atome d'hydrogène ou le groupe méthyle,
R² représente un atome d'hydrogène ou le groupe méthyle,
R³ est absent ou représente un radical alkylène en C₁-C₆, un radical cycloaliphatique en C₄-C₆ ou un radical arylène en C₆-C₁₂,
R⁴ représente un radical aliphatique en C₂-C₁₀ n-fois substitué, un radical cycloaliphatique en C₄-C₅, un radical aromatique en C₆-C₁₂, un radical hétérocyclique en C₄-C₁₂ qui peut contenir de 1 à 6 hétéroatomes, un reste saturé ou insaturé d'un composé phosphoré-azoté,
R⁵ est absent ou représente un radical alkylène en C₁-C₃,
X est absent ou représente O, -O-CO- ou -O-CO-NH-,
Y est absent ou représente O, -O-CO- ou -O-CO-NH-,
m vaut 0 ou 1 et
n vaut 3 ou 4.

3. Matériau dentaire selon la revendication 2, dans lequel au moins l'une des variables a l'une des significations suivantes :
R¹ représente un atome d'hydrogène ou le groupe méthyle,
R² représente un atome d'hydrogène ou le groupe méthyle,
R³ est absent ou représente un radical alkylène en C₁-C₆,
R⁴ représente un radical aliphatique en C₂-C₆ n-fois substitué, un radical aromatique en C₆-C₁₀, un reste d'acide cyanurique ou isocyanurique, qui peut contenir de 1 à 6 hétéroatomes, un reste triphosphazène cyclique,
R⁵ est absent,
X représente O ou -O-CO-NH-,
Y est absent ou représente O,
m vaut 0 ou 1 et
n vaut 3 ou 4.

4. Matériau dentaire selon l'une quelconque des revendications précédentes, qui en outre contient au moins un amorceur pour la polymérisation radicalaire.

5. Matériau dentaire selon l'une quelconque des revendications précédentes, qui en outre contient au moins un autre monomère polymérisable par voie radicalaire.

6. Matériau dentaire selon la revendication 5, qui contient un monomère polymérisable par voie radicalaire, comportant 2 ou 3 groupes susceptibles de polymérisation radicalaire.

7. Matériau dentaire selon l'une quelconque des revendications précédentes, qui en outre contient au moins un monomère polymérisable par voie radicalaire, contenant des groupes acides.

8. Matériau dentaire selon la revendication 7, dans lequel le monomère contenant des groupes acides contient en tant que groupe acide un groupe carboxy, un groupe phosphono, un groupe phosphate et/ou un groupe sulfo.

9. Matériau dentaire selon l'une quelconque des revendications précédentes, qui en outre contient au moins une charge.

10. Matériau dentaire selon l'une quelconque des revendications précédentes, qui contient
| | |
|---|---|
| 1 à 95 % en poids | de sulfure d'allyle selon la formule (I) ; |
| 0,01 à 5 % en poids | d'amorceur pour la polymérisation radicalaire ; |
| 0 à 60 % en poids | d'un autre monomère polymérisable par voie radicalaire ; |
| 0 à 20 % en poids | de monomère contenant des groupes acides ; |
| 0 à 85 % en poids | de charge. |

11. Matériau dentaire selon la revendication 10, pour utilisation en tant que composite pour obturation, qui contient
| | |
|---|---|
| 1 à 45 % en poids | de sulfure d'allyle selon la formule (I) ; |
| 0,01 à 5 % en poids | d'amorceur pour la polymérisation radicalaire ; |
| 0 à 50 % en poids | d'un autre monomère polymérisable par voie radicalaire ; |
| 30 à 85 % en poids | de charge ; |
| 0 à 10 % en poids | de monomère contenant des groupes acides. |

12. Matériau dentaire selon la revendication 10, pour utilisation en tant que ciment, qui contient
| | |
|---|---|
| 1 à 60 % en poids | de sulfure d'allyle selon la formule (I) ; |
| 0,01 à 5 % en poids | d'amorceur pour la polymérisation radicalaire ; |
| 0 à 60 % en poids | d'un autre monomère polymérisable par voie radicalaire ; |
| 20 à 60 % en poids | de charge ; |
| 0 à 15% en poids | de monomère contenant des groupes acides. |

13. Matériau dentaire selon la revendication 10, pour utilisation en tant que matériau de revêtement, qui contient
| | |
|---|---|
| 1 à 95 % en poids | de sulfure d'allyle selon la formule (I) ; |
| 0,01 à 5 % en poids | d'amorceur pour la polymérisation radicalaire ; |
| 0 à 60 % en poids | d'un autre monomère polymérisable par voie radicalaire ; |
| 0 à 20 % en poids | de charge ; |
| 0 à 10 % en poids | de monomère contenant des groupes acides. |

14. Matériau dentaire selon la revendication 10, pour utilisation en tant qu'adhésif, qui contient
| | |
|---|---|
| 1 à 80 % en poids | de sulfure d'allyle selon la formule (I) ; |
| 0,01 à 5 % en poids | d'amorceur pour la polymérisation radicalaire ; |
| 0 à 60 % en poids | d'un autre monomère polymérisable par voie radicalaire ; |
| 0 à 20 % en poids | de charge ; |
| 0 à 20 % en poids | de monomère contenant des groupes acides ; |
| 0 à 40 % en poids | de solvant. |

15. Utilisation d'un sulfure d'allyle selon la formule (I), pour la préparation d'un matériau dentaire.

16. Utilisation selon la revendication 15, pour la préparation d'un composite pour obturation, d'un ciment, d'un matériau de revêtement ou d'un adhésif.

17. Procédé pour la production de corps moulés durcis, dans lequel on met sous forme d'un corps moulé un matériau dentaire selon l'une quelconque des revendications 1 à 14 et ensuite on le fait durcir au moins partiellement.

18. Procédé selon la revendication 17, dans lequel le corps moulé est une couronne, un bridge, un inlay (incrustation), un onlay (incrustation de surface), une dent artificielle ou une autre restauration dentaire.
